# EUROPEAN PATENT APPLICATION

(11) **EP 1 529 508 A2**
(43) Date of publication of application: **11.05.2005**
(21) Application number: 04025668.7
(22) Date of filing: 28.10.2004
(51) Int. Cl.: A61F 13/15

(54) **Transparent absorbing article**

(30) Priority: 31.10.2003 US 699052
(71) Applicant: McNEILL-PPC, INC., Skillman, NJ 08558 (US)
(72) Inventor: Arora, Tarun K., Edison NJ 08817 (US); Gell, Carol B., Belle Mead NJ 08502 (US); Luizzi, Joseph M., Newtown PA 18940 (US); Moscherosch, H. Michael, Doylestown PA 18901 (US)
(74) Representative: Groening, Hans Wilhelm, Dipl.-Ing.

(57) **Abstract**

An absorbent article having a silhouette that includes a first portion and a second portion, wherein the second portion is in opposite relation to the first portion, and a pair of opposed longitudinally extending sides connecting the first portion to the second portion. The absorbent article also includes a layered portion with a substantially transparent, body-faceable cover layer, and a substantially transparent liquid-absorbing coating spaced apart from the substantially transparent liquid-permeable cover layer by a substantially transparent separating layer.

## Description

### Field of the Invention

This invention relates to absorbent articles, such as, pantyliners, sanitary napkins, incontinence pads, and the like. More particularly, the present invention relates to discrete absorbent articles adapted to be worn in a perineal region of the wearer.

### Background of the Invention

Disposable absorbent articles that having absorbent materials are disclosed in the literature and are commercially available. Typical disposable absorbent articles may include components such as (a) a body-faceable cover layer designed to keep the body dry, (b) an absorbent system that generally holds and contains the bulk of any bodily discharges (e.g., blood, menses, urine, etc.), and (c) a liquid-impermeable barrier layer that prevents any bodily discharges from leaking out of the absorbent article.

Typically, disposable absorbent articles used as sanitary protection are white. Because the materials, e.g., fibers and films, used to make the components, e.g., cover, absorbent core and barrier, of such absorbent articles often do not have the desired color, pigments, dyes, or other color imparting materials, such as, titanium dioxide, carbon black, and the like, are added to such materials to produce the desired color. However, for example, a cover and barrier layer produced from such pigmented materials may make the article or its contour highly visible, thereby reducing discretion.

For improved discretion, discrete absorbent article having one or more components or portions that are at least substantially transparent have been described in the literature. However, typically these articles offer only limited solution to the problem of discretion in that one or more portions or components of the article, as worn by the user are not substantially transparent, and therefore the article as a whole is not discrete. For example, these articles often include absorbent systems having materials that are white and therefore readily visible, thereby not supporting the transparency of the article.

Other discrete absorbent articles previously described have a plurality of transparent components or layers, including an absorbent core that supports the transparency of the article. However, to provide absorbency, the absorbent core includes absorbent gelling materials that are in the form of granules, fibers, or sheets, or strips. These absorbent cores suffer from drawbacks. For example, fibers of absorbent gelling materials are typically not cost-effective, sheets of absorbent gelling materials are difficult to manufacture, and granules are capable of shifting during transport and use of the article.

Other known methods of forming transparent articles include treating a substrate with a solution of polyacrylic acid oligomers that are crosslinked upon removal of solvent (e.g., water). Unfortunately, such solutions of polyacrylic acid oligomers are susceptible to microbial contamination, are relatively expensive, and have limited commercial availability.

Furthermore, such articles are not optimized for efficient absorbency across the lateral expanse or "silhouette" of the article. Accordingly, the need exists for an absorbent article that provides user discretion and overcomes one or more of the above-mentioned drawbacks.

### Summary of the Invention

The present invention provides an absorbent article having a silhouette that includes a first portion and a second portion, wherein the second portion is in opposite relation to the first portion, and a pair of opposed longitudinally extending sides connecting the first portion to the second portion. The absorbent article also includes a layered portion with a substantially transparent, body-faceable cover layer, and a substantially transparent liquid-absorbing coating spaced apart from the substantially transparent liquid-permeable cover layer by a substantially transparent separating layer.

In another aspect of the invention, a method of forming a substantially transparent absorbent article including: providing a substrate selected from the group consisting of a liquid-impermeable barrier layer, and a separating layer; applying a coating composition to the substrate to form a substantially transparent, liquid absorbing coating thereon; and positioning the substantially transparent, liquid absorbing coating such that is spaced apart from a liquid-permeable cover layer by the separating layer, thereby forming the substantially transparent article.

### BRIEF DESCRIPTION OF THE DRAWINGS

A more particular description of the invention that is briefly summarized above may be had by reference to the embodiments thereof that are illustrated in the appended drawings. It is to be so noted, however, that the appended drawings illustrate only typical embodiments of the invention and, therefore, are not to be considered limiting of its scope, for the invention may admit to other equally effective embodiments.
Figure 1 shows a top plan view of an absorbent article consistent with an embodiment of the invention described herein;
Figure 2A shows a cross-sectional view of the absorbent article of Figure 1 taken through line A-A;
Figure 2B shows a cross-sectional view of an alternative embodiment of the absorbent article of Figure 1 taken through line A-A;
Figure 2C shows a cross-sectional view of another alternative embodiment of the absorbent article of Figure 1 taken through line A-A; and
Figure 3 is a top plan view of an absorbent article consistent with another embodiment of the invention described herein.

### Detailed Description of the Invention

As used herein, all disclosed ranges expressly include and disclose all values between the endpoints.

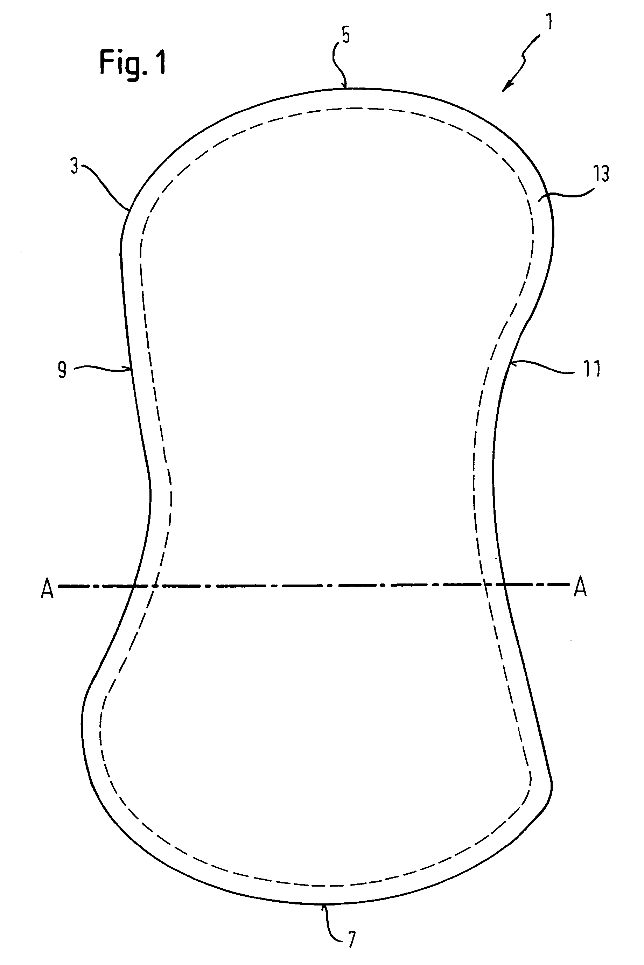

The absorbent articles of this invention, include, but are not limited to, sanitary napkins, pantyliners, incontinence devices, wound care articles, e.g., surgical dressings and adhesive bandages, shoe liners and the like. Figure 1 depicts an absorbent article 1, which may be, for example, a pantiliner, a sanitary napkin, an interlabial device, an adult incontinence device, or a diaper, of the present invention.

The absorbent article 1 has a silhouette 3 that generally defines the expanse of the absorbent article 1. The silhouette 3 includes a first transversely-extending edge 5 and a second transversely-extending edge 7 that is generally in opposite relation to the first transversely-extending edge 5. The silhouette 3 further includes a first longitudinally-extending edge 9 and a second longitudinally extending edge 11 that is generally in opposite relation to the first longitudinally-extending edge 9. The first longitudinally-extending edge 9 and the second longitudinally extending edge 11 connect the first transversely-extending edge 5 and the second transversely-extending edge 7. The absorbent article optionally includes flaps (not shown in the Figures), also known as wings, tabs and the like, that are generally flexible and configured to be folded over the edges of the underwear so that the flaps anchor or secure the absorbent article to the underwear. The design of the flaps is not critical. The flaps may include extensions of either or both of a barrier layer, a cover layer (the barrier layer and cover layer are discussed in detail below), or the cover and barrier layer adhered or laminated together.

The absorbent articles of the present invention can be of various shapes and configurations depending on the intended end use, e.g., sanitary protection, including pantyliners and sanitary napkins, surgical dressings or wipes, and the like. Additionally, the present absorbent articles can be incorporated into a disposable or limited use garment as an integral part thereof. For example, an absorbent article made according to the present invention can be a part of disposable training pants and similar garments. In one embodiment of the invention, the absorbent article is a sanitary napkin or pantiliner having a shape that is substantially rectangular, dog-bone-like, or peanut-like.

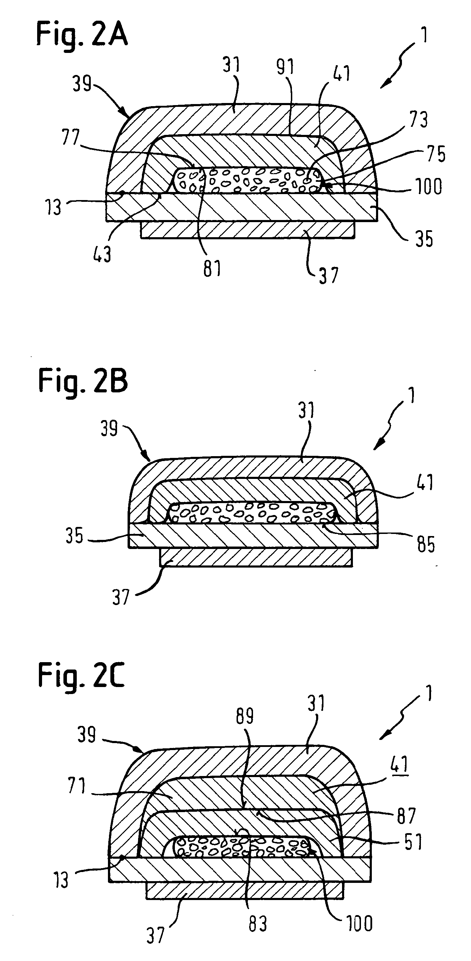

Figure 2A depicts a cross-sectional view of the absorbent article of Figure 1 taken through line A-A. The absorbent article 1 has a layered structure 39. In particular, the absorbent article 1 includes a body-faceable, fluid-permeable cover layer 31, a substantially transparent, liquid-absorbing coating 100 spaced apart from the cover layer 31, a separating layer 41, an optional fluid-impermeable, and a garment-faceable barrier layer 35. The cover layer 31 and the barrier layer 35 may be joined or sealed to each other along a peripheral edge 13 using any method known in the art. The cover layer 31 and the barrier layer 35 may encase the separating layer 41, the coating 100 as well as other layers that may be included in the article. Although not shown in Figure 1, the barrier layer 35, the cover layer 31, and optionally the separating layer 41 and the coating 100 may be extended to form flaps useful (particularly for panty liners, sanitary napkins, and the like) for folding about an edge of an undergarment.

The cover layer 31 is generally compliant, soft feeling, and non-irritating to the user's skin. The cover layer 31 generally functions to transport liquid away from the wearer into the absorbent article 1. In this manner, fluid and moisture are removed from contacting the wearer, thus making the wearer feel dry and comfortable. The cover may also possess low rewet potential, i.e., when, for example, pressure is applied to the absorbent article, absorbed liquid is not readily released and thus capable of "re-wetting" the wearer.

The cover layer 31 is substantially transparent. As used herein, "substantially transparent" refers to those individual layers or articles having a structure, composition, thickness, such that between about 45 to about 100 percent of light intensity in the visible spectrum is transmitted through the thickness of the layer of material or article. Total transmittance is the ratio of total transmitted light to incident light. It is reduced by reflectance and absorbance. The cover layer 31 may be substantially or completely free of colorants such as dyes and/or pigments (e.g., titanium dioxide, among other colorants) that are typically added to plastic films and fibers in order to impart a particular color (e.g., white, black, red, blue, green, among others) thereto.

In one embodiment of the invention, the cover layer 31 includes an interconnected network of woven and/or nonwoven fabrics formed from polyester, polypropylene, polyethylene, nylon, and/or rayon fibers or the cover layer 31. The fibers may be oriented by a carding process and thermally bonded via embossing. The cover layer 31 may include an apertured thermo-plastic film or a formed film. The fibers or other materials that make up the cover layer 31 should not collapse or lose their resiliency when subjected to body fluid. The cover layer 31 may optionally be treated with surfactant to manipulate the hydrophobicity/hydrophilicty thereof to facilitate optimal fluid transport properties. The thickness of the cover layer 31 may vary from about 0.025 mm to about 5 mm, depending on the material chosen. The basis weight of the body faceable cover layer 31 material may be between about 5 to about 150 grams per square meter (gsm). In one embodiment of the invention, the cover layer 31 has a basis weight less than about 75 gsm, less than about 30 gsm, or less than about 9 gsm.

One suitable example of an apertured thermo-plastic film that may be used for the cover layer 31 is unpigmented apertured film X28024, available from Tredegar Corporation of Richmond, Virginia. Suitable examples of a non-woven fibrous material that may be used for the cover layer 31 include unpigmented nonwoven (9 gsm), available from Shalag of Upper Tiberias, Israel, and unpigmented nonwoven (9 gsm) available from Cia Provicência Industria e Comercio of Curitiba, Brazil.

Spaced apart from the cover layer 31 is a substantially transparent, liquid-absorbing coating 100. The substantially transparent, liquid-absorbing coating 100 has a high capacity for absorbing bodily exudates (e.g., urine, menstrual fluid, etc.). When these liquids move "down" from the cover layer 3, they are conveyed to the liquid-absorbing coating 100, which retains the bulk of the liquid until the absorbent article 1 is discarded. The liquid-absorbing coating 100 is generally smaller (i.e., it occupies a smaller area when viewed from the top) than the barrier layer 35 and the cover layer 31.

The substantially transparent, liquid absorbing coating 100 may include a material capable of absorbing aqueous liquids and a matrix 75, such as a matrix of adhesive that surrounds the liquid absorbing material. The liquid-absorbing material is capable of absorbing and trapping liquid within the liquid absorbing material but is not soluble in the liquids absorbed. The liquid absorbing material may be discrete liquid-absorbing particles 73 that are distributed substantially homogeneously in the matrix 75.

The amount of liquid absorbing material will define the absorbency of the material; the more aqueous liquid absorbing material added, the greater the absorbency. In one embodiment of the invention, the matrix 75 is a hot melt adhesive and the coating 100 is a homogeneous mixture of greater than about 1% by weight of the liquid-absorbing material in the matrix 75. In another embodiment of the invention, the coating 100 includes greater than about 40% by weight of the liquid-absorbing material. In another embodiment of the invention, the coating 100 includes greater than about 60% by weight of the liquid-absorbing material.

The matrix 75 may include a block co-polymer. Suitable block copolymers for use in the invention include linear or radial co-polymer structures having the formula (A-B)ₓ, wherein block A is a polyvinylarene block, block B is a poly(monoalkenyl) block, x denotes the number of polymeric arms, and wherein x is an integer greater than or equal to one. Suitable block A polyvinylarenes include, but are not limited to polystyrene, polyalpha-methylstyrene, polyvinyltoluene, and combinations thereof. Suitable Block B poly(monoalkenyl) blocks include, but are not limited to, conjugated diene elastomers such as for example polybutadiene or polyisoprene or hydrogenated elastomers such as ethylene butylene or ethylene propylene or polyisobutylene, or combinations thereof. Commercial examples of these types of block copolymers include Kraton™ elastomers from Shell Chemical Company Vector™ elastomers from Dexco, Solprene™ from Enichem Elastomers and Stereon™ from Firestone Tire & Rubber Co. The matrix 75 is, in one embodiment of the invention, substantially free of water.

The matrix 75 may include a tackifying resin. Suitable tackifying resins include natural and modified resins; glycerol and pentaerythritol esters of natural and modified resins; polyterpene resins; copolymers and terpolymers of natural terpenes; phenolic modified terpene resins and the hydrogenated derivatives thereof; aliphatic petroleum resins and the hydrogenated derivatives thereof; aromatic petroleum resin and the hydrogenated derivatives thereof; and aliphatic or aromatic petroleum resins and the hydrogenated derivatives thereof, and combinations thereof. Commercial examples of these types of resins include Foral® hydrogenated rosin ester, Staybelite® hydrogenated modified rosin, Poly-pale® polymerized rosin, Permalyn® rosin ester, Pentalyn® rosin ester, Adtac® oil extended hydrocarbon resin, Piccopale® aromatic hydrocarbon, Piccotac®, Hercotac® aromatic modified aliphatic hydrocarbon, Regalrez® cycloaliphatic resins, or Piccolyte® from Hercules, Eselementz® from Exxon Chemical aliphatic hydrocarbon and cycloaliphatic resins, Wingtack® from Goodyear Tire & Rubber Co. synthetic polyterpene resins including aromatic modified versions, Arkon® partially and fully hydrogenated aromatic resins from Arakawa Chemicals, Zonatac® styrenated terpene resin, Zonarez® rosin ester and Zonester® rosin ester from Arizona Chemical and Nevtac® aromatic modified aliphatic hydrocarbon from Neville Chemical Company.

Liquid-absorbing materials suitable for inclusion in the coating 100 include thermoplastic hydrogels such as superabsorbent materials or thermoplastic polymeric compositions, which are, for example, formed from a water-soluble soft segment and one or more hard segments. The hard segment may be melt processable, i.e., at use temperature the hard segments in the polymer are below their melt temperature, and at process temperature, the hard segments are above their melting point temperature and below the decomposition temperature of either the other components of the hot-melt adhesive composition. The hard segment is substantially insoluble in water, and phase separates from the soft segment. Examples of suitable hard segments include, but are not limited to polyurethane, polyamides, polyesters, polyureas, and combinations thereof. Examples of suitable soft segments include, but are not limited to polyethylene oxide, polyvinyl alcohol, polyvinyl pyrrolidone, polyacrylamide, polysaccharide, polymaleic anhydride, random copolymers of polyethylene oxide and polypropyleneoxide and combinations thereof. The soft and hard segments may be covalently bonded together by means of urethane, amide, ester, or secondary urea linkages or combinations thereof. Examples of aqueous liquid absorbing thermoplastic polymeric compositions which are commercially available include hydrophilic polyurethane from Tyndale Plains-Hunter Ltd. and Aquacaulk® thermoplastic polymers from Sumitomo Seika Chemicals Co., Ltd. Suitable superabsorbent materials include any of the conventional superabsorbent particles or superabsorbent fibers which are commercially available today. Examples are Aquakeep SA-70 and J-550P from Sumitomo Seika Chemicals Co., Ltd.

For embodiments of the invention in which the liquid-absorbing material is present as liquid absorbing particles 73, such as superabsorbent particles, the particles 73 may be present in units having a dimension/diameter less than about 150 microns. In one embodiment of the invention, the particles 73 have an average particle size less than about 125 microns. In another embodiment of the invention, the particles 73 have an average particle size between about 10 microns and about 75 microns. The small particle size of the liquid absorbing material results in higher surface area of the liquid absorbing particles 73 and therefore higher liquid absorbency. Furthermore, the small particle size also facilitates attaining a homogeneous mixture and ease of processing the material through conventional hot melt adhesive application equipment.

The matrix 75 may include a plasticizer. Suitable plasticizers for use in the present invention include any conventional plasticizers which decrease hardness and modulus, enhance pressure sensitive tack and reduce melt and solution viscosity. It is preferred that the plasticizer be water soluble or water dispersible or alternatively be a wax-like substance such as polyethylene glycol, glycerin, glycerol, polypropylene glycol, butylene glycol or sorbitol. An example of a preferred plasticizer is Carbowax® polyethylene glycol from Union Carbide.

The matrix 75 may include additional functional ingredients. For example, the coating 100 may include an anti-oxidant. Suitable anti-oxidants for use in the present invention include any conventional anti-oxidants, such as hindered phenols, for example, Ethanox 330w 1,3,5-trimethyl-2,4,6-tris(3,5-di-tert-butyl-4-hydroxybenzyl) benzene which is commercially available from the Ethyl Corporation.

In one embodiment of the invention, the coating 100 includes (by weight): about 10 - about 50% block copolymer (such as block copolymer having a melt index greater than about 10), about 20 - about 80% tackifying resin (such as having a softening point less than about 105°C.), greater than about 1% liquid absorbing polymer, about 0 - about 40% plasitcizer with viscosity from about 1 - about 500 centipoise at application temperature, and about 0 - about 2.0% antioxidant.

The coating 100 may be substantially free of fibrous material, i.e., in this case it is substantially free of fibers such as synthetic, cellulosic, and other fibers that are processed using traditional fiber processing techniques (air-laid, wet-laid, carding, and the like) and commonly used in transfer layers and absorbent cores of absorbent articles.

The substantially transparent, liquid-absorbing coating 100 is spaced apart from the cover layer 31 by a substantially transparent fluid management medium, such as the separating layer 41. The separating layer 41 generally serves to separate the cover layer 41 from the liquid-absorbing coating 100. The separating layer 41 also may serve to provide lateral wicking of liquid that is transmitted through the cover layer 31, thereby providing greater utilization across the silhouette 3 of the article 1 as well as reduction of gel-blocking within the coating 100. Alternatively, or in addition to wicking benefits, the separating layer 41 may also serve to provide a substrate upon which the liquid absorbing coating 100 is deposited. The separating layer 41 may serve either or both of these functions in one unitized layer. Alternatively, either or both of these functions may be performed by the separating layer 41 that includes a discrete transfer layer for distributing fluid and/or a discrete insert that serves to provide a substrate upon which the liquid absorbing coating 100 may be deposited. In addition to serving either or both of the functions of fluid distribution and/or substrate, the separating layer 41 may also function to absorb and contain bodily fluids. The separating layer 41 is substantially transparent.

The separating layer 41 may have a thickness in a range from about 0.5 mm to about 3.0 mm. The transfer layer may have a bulk density in a range from about 0.03 g/cc to about 0.15 g/cc. Furthermore, the separating layer 41 has a basis weight that is high enough to maintain its mechanical integrity during use of the product, including when the separating layer 41 is wet with bodily exudates. The separating layer 41 may have, for example, a basis weight in a range from about 5 gsm to about 200 gsm.

Generally, with other attributes (e.g., thickness, fiber denier, materials of construction, and the like) of the separating layer held constant, the transparency of the separating layer 41 decreases with basis weight. However, it is possible to maintain substantial transparency (e.g., greater than about 45%) of the separating layer 41 even if the basis weight of the transfer layer is high, by increasing the open area (i.e., the void area within the separating layer 41 that is unoccupied by fibers) in the layer. Increasing open area may be accomplished by, for example, increasing the specific gravity of the material that the fibers are formed from, decreasing the denier of the fibers, and the like. In one embodiment of the invention, the denier of the fibers in the transfer layer is between about 1.5 denier per fiber (dpf) and about 15 dpf; such as between about 6 dpf and about 15 dpf. These ranges of denier may be suitable, for example, when the separating layer 41 is an aggregate of polypropylene fibers having a basis weight of about 45 gsm. In one embodiment of the invention, to enhance light transmission, the separating layer 41 is free of cellulose, such as cellulose wood pulp, and the like, although this absence of cellulose is not required.

In one embodiment of the invention, the separating layer 41 is a transfer layer capable of distributing fluid in a direction generally parallel to the cover layer 31. As such, fluid impinging upon the cover layer 31 is spread across the silhouette 3 of the absorbent article, providing efficient management of fluid. In this embodiment of the invention, the transfer layer generally has sufficient thickness or loft to prevent fluid from penetrating through to a back surface 43 of the transfer layer before the fluid has a sufficient opportunity to spread outward. The transfer layer receives body fluid from the fluid-permeable cover layer 31 and holds it until the substantially transparent liquid-absorbing layer 100 has an opportunity to absorb it. The transfer layer is generally more dense than the cover layer 31 and has a larger proportion of smaller pores than does the cover layer 31. These attributes allow the transfer layer to contain body fluid and hold it away from the outer side of the cover layer 31, thereby preventing the fluid from re-wetting the cover layer 31 and its outer surface. However, the transfer layer is preferably not so dense as to prevent the passage of the fluid through the transfer layer and into the underlying liquid-absorbing layer 100.

The transfer layer may include various materials, including, for example, fibrous webs, resilient foams, and the like. The transfer layer may include synthetic polymer materials such as single component or bicomponent fibers that include thermoplastic materials (such as polyester, polypropylene, polyethylene, polyethylene terepthalate, among others) in fiber or other forms, rayon, organic binders (such as copolymers of vinyl, acrylic and/or other monomers that may be coated onto thermoplastic fibers or otherwise incorporated into the transfer layer) among other materials known to the art.

The transfer layer may include hydrophobic, nonabsorbent fibers that are able to accept large volumes of fluid into interfiber void spaces while the fibers themselves need not absorb any significant quantities of fluid. Likewise, open-celled foam structures that are made from nonabsorbent materials may also absorb fluid into the cells of the foam. The walls of the cells, however, need not absorb any fluid. The cumulative spaces within the transfer layer, i.e., the interfiber void spaces in the fibrous transfer layer or the open cells in the foam transfer layer, function much like a container to hold fluid.

The transfer layer may be a fibrous web comprising resilient, nonabsorbent materials to provide void volume and to allow for free movement of fluid through the structure. Transfer layers that are made from webs of mostly absorbent fibers absorb the fluid as it enters the structure and do not distribute it throughout the rest of the structure as efficiently as webs containing non-absorbent materials. In one embodiment of the invention, the transfer layer includes an unpigmented apertured plastic film, such as Tredegar AF X28024, having a basis weight of, for example, about 24 gsm.

The coating 100 may be coated on a bottom surface 81 of the separating layer 41 as shown in Figure 2A. Alternatively, the coating 100 may be coated upon a top surface 85 of the barrier layer 35, as shown in Figure 2B.

The separating layer 41 may be a multi-layer structure as shown in Figure 2C. In this embodiment of the invention, the separating layer 41 includes a transfer layer 71 and an insert 51. The insert 51 acts as a substrate upon which the liquid-absorbing coating may be deposited. The insert 51 may provide additional fluid-distribution properties to enhance the fluid transfer or acquisition properties of the transfer layer 71, or the insert 51 may provide liquid retention (absorbing) properties to enhance those of the liquid-absorbing coating 100. While Figure 2C shows the coating 100 formed on a bottom surface 83 of the insert 51, the coating 100 may alternatively be formed on a top surface 87 of the insert 51 or a bottom surface 89 of the transfer layer 71.

Note that various gaps 91 are shown in Figures 2A-2C to illustrate, even if construction adhesive is used to bond one or more of the layers together, that the various layers of the article 1 need not conform exactly to one another. These gaps 91, are substantially void of material may, for example, be on the order of about 0.05 mm to about 1.5mm as the article 1 is laid flat in an uncompressed state. Note that no gaps 91 are depicted in Figures 2A-2C between the coating 100 and the surface upon which it is coated. This is because the coating 100 is generally intimately bonded to the layer upon which it is coated (i.e., its substrate) and no substantial spacing is present between the coating 100 and its substrate.

The insert 51 may be a thin layer of fibrous material that retains fluid and is positioned intermediate the transfer layer 71 and the substantially transparent, liquid-absorbing coating 100 or intermediate the substantially transparent, liquid-absorbing coating 100 and the barrier layer 35. Examples of material that may be used in the construction of the insert 51 include, for example, cellulosic fibers (e.g., wood pulp, cotton or flax), synthetic fibers, superabsorbent polymers (SAP) or superabsorbent fibers, as well organic binders or other materials described above as suitable for incorporation into the transfer layer, and other materials known to the art of manufacturing absorbent core materials. The relative proportion of these materials may be varied to achieve sufficient absorbency, compressibility, and processibility. Such highloft webs may be bonded with chemical binders or by thermal means such as by through-air bonding. The insert 51 may be, for example, a layer of high-loft non-woven material. One suitable example includes a layer of thermal bonded network of polypropylene fibers (e.g., 45 gsm), such as is available from Polystar Industria e Comercio de Produtos Sinteticos LTDA of Salvador, Brazil. The insert 51 may be resin or thermal bonded, and flat or emboss calendared to achieve an appropriate strength. The density of the insert 51 may be in a range from about 0.08 g/cc to about 0.60 g/cc.

The absorbent article 1 may also include a garment faceable, liquid-impermeable barrier layer 35. The barrier layer 35 is substantially transparent and impermeable to liquids and, thus, prevents bodily fluids from soiling the clothing of the user. Suitable materials that may be incorporated into the barrier layer 35 include, for example, embossed or non-embossed polyethylene films, microporous films, and laminated tissue, among other materials. In one embodiment of the invention, the barrier layer 35 layer is a thin, flexible, fluid impermeable material, such as, a polymeric film, e.g., polyethylene, polypropylene, or cellophane, or a normally liquid permeable material that has been treated to be impermeable, such as, impregnated fluid repellent paper or non-woven material, including non-woven fabric material, or a flexible foam, such as polyurethane or cross-linked polyethylene. The garment faceable, liquid-impermeable barrier layer 35 is substantially transparent and may be free of colorants such as dyes and/or pigments. The thickness of the barrier layer when formed from a polymeric film may be about 0.025 mm to about 0.051 mm. One suitable example of material that may be used for the barrier layer 35 is Tredegar (non-shine) X28480, available from Tredegar Film Products. The barrier layer 35 may include ethylene vinyl acetate (EVA), although EVA is not required.

The garment faceable, liquid-impermeable barrier layer 35 may be breathable, i.e., intended to allow the passage of vapor or water molecules through them while retarding, at least to a degree, the passage of fluid. Monolithic film technology provides materials that can be used to form sheets that allow vapor transmission, but are relatively impermeable to liquids. Commonly used materials are segmented block copolymers, polyvinyl alcohol, polyurethane, cellulosics and blends thereof. Other materials that may be used as impermeable barriers may be chosen from films of polypropylene, polyethylene, polyesters, polyamides, polyethylene vinyl acetate, polyvinyl chloride, and polyvinylidene chloride. Co-extruded and laminated combinations of the foregoing, wherein such combinations are permitted by the chemical and physical properties of the film, may be used. Fluid impermeable nonreticulated foams may also be used.

A suitable barrier layer 35 material may be a pigment-free monolithic film made from segmented block copolymers where one block is hydrophilic and the other block hydrophobic in nature, alternative materials for monolithic films are polyvinyl alcohol, polyurethane, cellulosics and blends thereof. In one embodiment of the invention, the barrier layer 35 is made from a pigment-free and filler-free monolithic sheet made from segmented block copolymers. For example, the backsheet may be 0.8 mil, polypropylene film such as that supplied by the Edison Plastic Co. (Newport News, VA) (Code XP-766-B), a film from Filmtech. Corp. (Lehigh Valley, PA), or it may be a polyethylene film available from Tredegar Film Products of Richmond, VA (Code X28480).

Note that various layers of the article 1 may be secured to one another using any suitable construction adhesive known to the art of absorbent article manufacture, to form a cohesive unit to enhance the article's stability. Such attachment or adherence may be by any known means, including, for example, adhesive, ultrasonics, co-embossing, thermobonding, mechanical bonding, and the like. However, it is preferred that the construction adhesive is colorless, e.g., a Gardner color of about 3 or less, and does not inhibit the light transmission, vapor transmission, or breathability of the barrier layer 35. The construction adhesive serves to hold the layers together and to minimize deformation during use. The adhesive can be applied as either a thin porous film or in a random spray, in a controlled spiral pattern, or in any other application pattern. See, for example, U.S. Patents Nos. 5,462,538; 5,681,305 and 5,885,681.

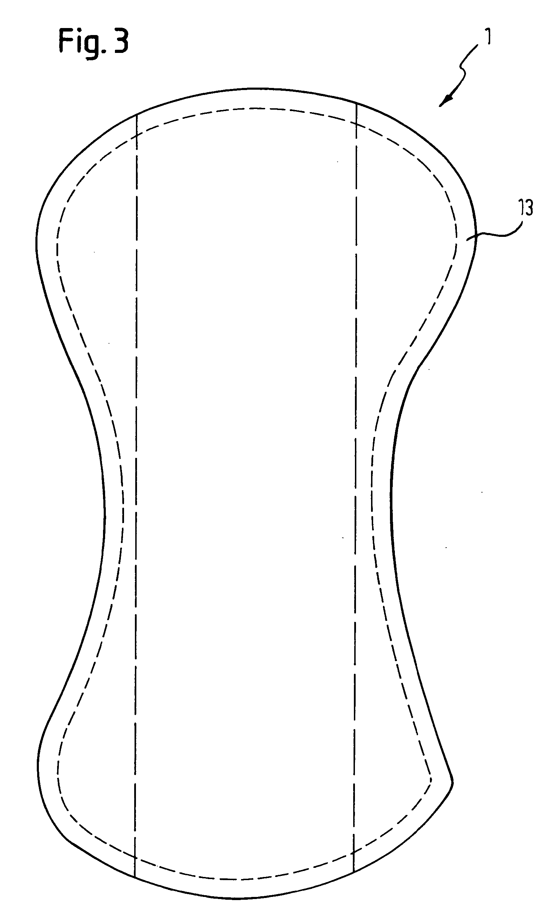

In one embodiment of the invention, as shown in Figure 3, the liquid absorbing coating 100 is co-extensive with the first longitudinally-extending edge 5 and/or the second longitudinally-extending edge 7. For example, as indicated in Figure 3, the coating 100 may extend continuously from the first longitudinally-extending edge 5 and the second longitudinally-extending edge 7. Furthermore, other layers that serve to absorb or transfer fluid such as the separating layer 41 (e.g., one or more of its components: the insert 51 and the transfer layer 71 may also be co-extensive with edges 5, 7.

Conventional articles that include superabsorbent materials (e.g., superabsorbent polymer) positioned proximate or in contact with the edge 13 are unacceptable weakened when the superabsorbent absorbs liquid, causing a rupture of the seal along the edge 13, and delamination of the article 1 (see Comparative Example 1 below). In one embodiment of the invention, the article 1 has a wet seal strength that is greater than about 80 grams/millimeter (g/mm), greater than about 100 g/mm, or greater than about 150 g/mm.

Referring again to Figure 2A-2C, the absorbent article 1 may include a securing element 37 for securing the absorbent article to the inside surface of the crotch portion of a garment. The securing element 37 may include, for example, adhesive, mechanical attachment devices, such as, clips, laces, ties, and interlocking devices, e.g., snaps, buttons, VELCRO (available from Velcro USA, Inc., Manchester, NH), zipper, and combinations of thereof. The securing element 37 may be deposited on the barrier layer 35, as shown in Figure 2A-2C.

Suitable adhesive that may be used as the securing element 37 may include pressure-sensitive adhesive, which may be applied as continuous or intermittent patches, strips, swirls, or waves, and the like. As used herein, the term pressure-sensitive adhesive refers to any releasable adhesive or releasable tenacious means. Suitable adhesive compositions, include, for example, water-based pressure-sensitive adhesives such as acrylate adhesives; emulsion or solvent-borne adhesives of polyisoprene, styrene-butadiene, or polyacrylate, vinyl acetate copolymer or combinations thereof; hot melt adhesives based on suitable block copoylmers; among other adhesive compositions now known or developed in the future in the art of absorbent article manufacture. In one embodiment of the invention, the pressure sensitive adhesive is free of colorants. The pressure sensitive adhesive may be applied to the outer surface barrier layer 35 of the absorbent article to help maintain it in place. As used herein, the term "pressure-sensitive adhesive" refers to any pigment-free releasable adhesive having a Gardner color of about 3 or less or releasable tenacious means that does not substantially decrease light transmissibility. Suitable pressure sensitive adhesives include, for example, water-based adhesives such as acrylate adhesives. Alternatively, the adhesive may be a rapid setting thermoplastic "hot melt" rubber adhesive or two-sided adhesive tape.

A paper release strip that has been coated on one side, may be applied to protect the adhesive on the barrier layer prior to use. The coating on the release paper, for example, silicone, reduces adherence of the coated side of the release to the barrier layer adhesive. The release strip can be formed from any suitable sheet-like material that, when coated, adheres with sufficient tenacity to the adhesive to remain in place prior to use, but can be readily removed when the absorbent article prior to placement on the wearer's underpants.

The article 1 has an overall thickness (measured in a relaxed, uncompressed state) that depends upon the particular use. For example, a pantiliner consistent with embodiments described herein may have a thickness greater than about 0.8 millimeters (mm). The overall thickness may be greater than 0.8 mm greater, such as greater than about 1 mm. The absorbency of the article 1 is such that the total capacity may be greater than about 2 grams. For a method of measuring total capacity, the reader is referred to U.S. patent 4,950,264, issued to the Procter & Gamble Company. The article 1 as a whole (with any release strip that is present, removed) is substantially transparent, i.e., the entire article has a transparency that is greater than about 45%.

The absorbent article, in the case of a pantyliner or sanitary napkin, may be applied to the crotch of underpants by removing any release strip and placing the barrier layer of the absorbent article against the inside of the crotch of the underpants. Other absorbent articles also within the scope of this invention also include wound care articles such as bandages, including adhesive bandages. Adhesive bandages usually have a barrier layer of perforated plastic or of a woven or knit fabric. The barrier layer is covered completely or partially on one side with a pressure sensitive adhesive. An absorbent core is placed in the center of and adhered to the adhesive side of the backing material. The absorbent core typically lies between a cover, which contacts the skin and prevents the absorbent from sticking to the wound, and the barrier layer. Alternately, absorbent articles within the scope of this invention also include shoe liners. Shoe liners usually have a non-skid backing and an absorbent.

Articles of the present invention may be fabricated by a variety of methods known to the art of absorbent article manufacture. For example, individual webs (expanses of material layers that are unrolled to expose a section to be processed) of material may be provided along, for example, a conveyer. The webs may include separate webs of the barrier layer 35, the cover layer 31, and the at least one separating layer 41.

A coating composition that includes, for example, liquid-absorbing polymer particles dispersed within an adhesive is coated upon one or more material layers that will constitute an absorbent article. The liquid absorbing polymer particles may have a particle size similar to those described previously. The composition may be formed, for example, by blending an adhesive that includes about 10% to about 50% of a block copolymer, and about 20% to about 80% of a tackifying resin, with greater than about 1% of a liquid absorbing polymer in suitable adhesive processing equipment such as a melt mixer or extruder at a temperature above the melting points of the constituents until they are uniformly mixed. An exemplary suitable hot melt composition is code NW-1078, commercially available from HB Fuller Co., St. Paul, MN.

The one or more material layers to be coated are selected from the barrier layer 35, the separating layer 41 (e.g., the transfer layer 71 and/or the insert 51). The coating composition may be heated from a non-flowable state to a temperature sufficient to render the composition flowable. The coating composition is deposited or formed on the one or more layers using, for example, conventional adhesive processing equipment such as a hot melt adhesive slot coating head, a hot melt adhesive swirl spray applicator (a commercial example of which is a Nordson Control Fiberization®), a hot melt adhesive micro fiber applicator (commercial examples of these applicators include Nordson Control Coat®, ITW Dynafiber®, J&M Meltblown, and May Coating's Accufiber®), a hot melt adhesive rotary screen applicator to create a pattern coating (examples of this equipment include Nordson and Kraemer rotary screen technology). For embodiments in which the substrate is compressible (e.g., nonwoven or other fibrous substrates), a control coating apparatus may be used to deposit the coating to maintain the thickness and loft of the substrate upon which the composition is coated. The deposition of the coating composition on the substrate upon cooling to ambient temperature, results in the formation of the substantially transparent coating 100 on the substrate.

The coating 100 is positioned in a spaced apart manner from the cover layer 31. The various layers are bonded together, such as by bonding the cover layer 31 to the barrier layer 35 along the edge 13. The various webs of materials may be bonded together using a suitable construction adhesive. The various webs may then be sealed together along a pattern similar to edge 13 shown in Figure 1. Application of tabs, positioning adhesive, release strips, among other films, layers, or structures may be performed as is known to the art of absorbent article manufacture.

Embodiments of the invention described herein possess one or more of the following advantages: a high degree of liquid absorbency, a high degree of transparency, the ability to separately vary transparency and absorbency by varying the thickness and/or materials that are included in the coating 100, efficient utilization of the silhouette 3 of the article 1 by varying the thickness and/or materials that are included in the separating layer 41, consistent and substantially uniform absorbency due to the absorbent material being substantially immobile within the article, and improved wet seal strength. The transparent, liquid-absorbing coating 100 may enable the article 1 to be more transparent than conventional articles that include cellulose-based absorbent layers, because the coating 100 generally offers a higher degree of absorbency without compromising transparency. Embodiments of the invention described herein may also provide manufacturing flexibility and simplicity in that the coating 100 may be co-extensive with the edges 5, 7 without rendering the article 1 vulnerable to a failure of the seal along the edge 13 during use.

After the invention has been described in general hereinbefore, the following example is intended to illustrate details of embodiments of the invention, without thereby limiting it in any matter.

### EXAMPLE A

Two absorbent articles were made and measured. The absorbent articles contained the following components:

| | **Comparative Example 1** | **Example 1** |
|---|---|---|
| | **(COMP 1)** | **(EX 1)** |
| Cover | Shalag nonwoven, 9 gsm, | Shalag nonwoven, 9 gsm, |
| | free of TiO₂ | free of TiO₂ |
| | (Code No. SH-PPR-09) | (Code No. SH-PPR-09) |
| | | |
| Transfer Layer | Tredegar polyolefin | Tredegar polyolefin apertured |
| | apertured film (Code No. | film (Code No. X28024), 24 gsm |
| | X28024), 24 gsm | |
| Transparent | ------ | 30 gsm superabsorbent adhesive |
| Absorbent Coating | | coated on insert |
| Insert | Polyester (Libeltex T-21) | 45 gsm Polystar nonwoven |
| | treated with 95 % Fullatex | |
| | PD 8081 H + 5% Bacote | |
| | 20 | |
| | | |
| Laminating | Fuller block co-polymer | Fuller block co-polymer hot melt |
| Adhesive | hot melt HL 1491 (7 gsm) | HL 1491 (7 gsm) |
| | | |
| Barrier Layer | Tredegar polyolefin film | Tredegar polyolefin film (code |
| | (code 28480), 24 gsm | 28480), 24 gsm |
| Positioning | Fuller block co-polymer | Fuller block co-polymer hot melt |
| Adhesive | hot melt HL 1417 (19 | HL 1417 (19 gsm) |
| | gsm) | |

The articles (COMP 1 and EX 1) were sealed at a seal temperature of 280 degrees Fahrenheit, under a pressure of 90 pounds for a dwell time of 7 seconds. Percent total transmittance was determined using a Gardner haze guard plus instrument model number 238 013 796K (BYK-Gardner USA, Columbia, MD), the transparency of the article was measured. A flat sample was placed in a round sample holder (approximately 60 mm diameter). Measurements were taken by placing the flat sample in the appropriate measuring ports of the instrument (haze port was used for transmittance and the haze, clarity port was used for clarity). A series of five readings were taken and averaged to obtain final measurement value. During the measurement, the narrow angle scattering was less than 2.5%. The wide angle scattering was greater than 2.5%. Total transmittance was calculated as the ratio of the total light transmitted to incident light. Total transmittance is reduced by reflectance and absorbance. The standard deviations for all measurements were less than 1.

The transmittance of light through the articles was measured. The total transmittance for COMP 1 was calculated as 63.8%. The total transmittance for EX 1 was calculated as 64.1%

Secondly, wet seal strength was determined using an Instron Stress Strain Apparatus with charting capability. The following parameters were selected: gauge length of ¾ inch, crosshead speed and chart speed of 12 inches per minute, fail rate of 100% fail. The instrument was allowed to warm up for 20 minutes and was calibrated as per the manufacturers instructions. Test samples were prepared by cutting fourteen 1" wide rectangular sections. Each section included a portion of the seal along the peripheral edge. Each sample was soaked for 15 seconds in water and placed on paper towels to remove excess moisture. The barrier layer was peeled apart from the cover layer to no less than 1/8" from the seal at the peripheral edge. The seal was placed in tension by clamping either end to the instrument, to the point of failure. Peak load was reported for each of the fourteen samples and averaged to determine wet seal strength. COMP 1 had a wet seal strength of 29 grams. EX 1 had a wet seal strength of 414 grams.

### EXAMPLE B

Five absorbent articles were measured for transparency. The absorbent articles are identified as follows:

| | |
|---|---|
| Comparative Example 2 (COMP 2) | CARE-FREE PERFECT FIT (Johnson & Johnson Consumer Products Companies of New Brunswick, NJ) |
| Comparative Example 3 (COMP 3) | SOFY Ultra Thin Long Pantiliner (Unicharm Corporation of Kawanoe shi, Japan) |
| Comparative Example 4 (COMP 4) | KNH Carnation butterfly-shaped ultra thin (KNH of Taipei, Taiwan) |
| Comparative Example 5 (COMP 5) | BARELY THERE (Johnson & Johnson Consumer Companies, Manila, Philipines) |
| Comparative Example 6 (COMP 6) | Whisper ultra thin breathable pantiliner, Unscented (Procter & Gamble India of Mumbai, India) |

The articles identified above (Example B) were evaluated for transparency (total transmittance) as described above for Example A. COMP 2 had a total transmittance of 30.5%. COMP 3 had a total transmittance of 28.0%. COMP 4 had a total transmittance of 42.5%. COMP 5 had a total transmittance of 23.4%. COMP 6 had a total transmittance of 22.0%.

## Claims

1. An absorbent article comprising:
A. a silhouette comprising
i. a first transverse edge,
ii. a second transverse edge, wherein the second transverse edge is in opposite relation to the first transverse edge, and
iii. a pair of opposed longitudinally-extending edges connecting the first transverse edge and the second transverse edge, and
B. a layered structure comprising
i. a body-faceable, liquid-permeable cover layer;
ii. a substantially transparent separating layer;
iii. a substantially transparent, liquid-absorbing coating spaced apart from the substantially transparent, liquid-permeable cover layer by the substantially transparent separating layer.

2. An absorbent article of claim 1 wherein the substantially transparent separating layer comprises a transfer layer.

3. An absorbent article of claim 1 further comprising a garment-faceable, liquid-impermeable barrier layer.

4. An absorbent article of claim 1 wherein the substantially transparent separating layer is cellulose-free.

5. An absorbent article of claim 1 wherein the separating layer comprises a material selected from the group consisting of an apertured plastic film, a non-woven material, a polymeric foam material, and combinations thereof.

6. An absorbent article of claim 1 wherein the separating layer has a thickness in a range from about 0.5 mm (millimeters) to about 3 mm.

7. An absorbent article of claim 1 wherein the separating layer has a basis weight in a range from about 5 grams per square meter (gsm) to about 200 gsm.

8. An absorbent article of claim 1 wherein the substantially transparent, liquid-absorbing coating is formed on a surface of the separating layer.

9. An absorbent article of claim 3 wherein the substantially transparent, liquid-absorbing coating is formed on a body-facing surface of the liquid-impermeable barrier layer.

10. An absorbent article of claim 3 wherein a layer selected from the group consisting of the liquid-permeable cover layer, the substantially transparent liquid-absorbing coating, the substantially transparent, liquid-impermeable barrier layer, the one or more substantially transparent separating layers, and combinations thereof is free of colorants.

11. An absorbent article of claim 10 wherein each of the liquid-permeable cover layer, the substantially transparent liquid-absorbing coating, the liquid-impermeable barrier layer, the one or more substantially transparent separating layers are free of colorants.

12. An absorbent article of claim 1 wherein the substantially transparent liquid-absorbing coating comprises a plurality of liquid absorbing particles.

13. An absorbent article of claim 1 wherein the substantially transparent liquid-absorbing coating comprises a mixture of a hot melt adhesive and a liquid-absorbing polymer.

14. An absorbent article of claim 12, wherein the liquid-absorbing particles comprise a superabsorbent polymer.

15. An absorbent article of claim 12, wherein the liquid-absorbing particles have an average diameter of less than about 150 microns.

16. An absorbent article of claim 12 wherein the substantially transparent liquid-absorbing coating comprises liquid-absorbing polymer particles having an average diameter of from about 10 microns to about 75 microns.

17. An absorbent article of claim 12, wherein the matrix comprises a block co-polymer and a tackifying resin.

18. An absorbent article of claim 17, wherein the block copolymer comprises a block selected from the group consisting of conjugated diene elastomers, hydrogenated elastomers, and combinations thereof.

19. An absorbent article of claim 1 wherein the transparent, liquid-absorbing coating has a basis weight greater than about 20 gsm.

20. An absorbent article of claim 1 wherein the transparent, liquid-absorbing coating is substantially free of fibrous material.

21. An absorbent article of claim 1 wherein the article has a light transmittance of greater than about 45%.

22. An absorbent article of claim 1 wherein the separating layer comprises a fibrous material having a denier in a range from about 1.5 denier per fiber (dpf) to about 15 dpf.

23. An absorbent article of claim 1 wherein the transparent, liquid-absorbing coating extends from the first longitudinally-extending edge of the article and the second longitudinally-extending edge of the article.

24. An absorbent article of claim 1 wherein the body-faceable, liquid-permeable cover layer is substantially transparent.

25. An absorbent article of claim 3 wherein the garment-faceable, liquid-impermeable barrier layer is substantially transparent.

26. An absorbent article of claim 11 wherein the garment-faceable, liquid-impermeable barrier layer is substantially transparent.

27. An absorbent article comprising:
A. a silhouette comprising
i. a first transverse edge,
ii. a second transverse edge, wherein the second transverse edge is in opposite relation to the first transverse edge,
iii. a pair of opposed longitudinally-extending edges connecting the first transverse edge and the second transverse edge; and
B. a layered structure comprising
i. a substantially transparent, body-faceable, liquid-permeable cover layer; and
ii.. a substantially transparent, liquid-absorbing coating spaced apart from the substantially transparent cover layer by a substantially transparent separating layer, wherein the substantially transparent, liquid-absorbing coating comprises a superabsorbent polymer dispersed in a matrix, and wherein the matrix comprises a thermoplastic hot melt-adhesive.

28. A method of forming a substantially transparent absorbent article, wherein the method comprises:
providing a substrate consisting of a liquid-impermeable barrier layer or a separating layer;
applying a coating composition to the substrate to form a substantially transparent, liquid absorbing coating thereon; and
positioning the substantially transparent, liquid absorbing coating such that the substantially transparent, liquid absorbing coating is spaced apart from the liquid-permeable cover layer by the separating layer.

29. A method of claim 28 further comprising heating the coating composition from a non-flowable ambient state to a temperature sufficient to render the composition flowable.

30. A method of claim 28 wherein the coating composition comprises liquid absorbing polymer particles.
